# EUROPEAN PATENT SPECIFICATION

(11) **EP 1 867 664 B1**
(45) Date of publication and mention of the grant of the patent: **30.11.2011**
(21) Application number: 06729365.4
(22) Date of filing: 17.03.2006
(51) Int. Cl.: C08F 220/06, A61Q 1/10

(54) **COSMETIC FOR EYELASH**
KOSMETIKUM FÜR AUGENWIMPER
COSMETIQUE POUR CILS

(30) Priority: 18.03.2005 JP 2005080515; 06.04.2005 JP 2005110224
(43) Date of publication of application: 19.12.2007
(73) Proprietor: Kao Corporation, Chuo-ku Tokyo 103-8210 (JP)
(72) Inventor: YOSHIDA, Kensuke, unka 2-chome, Sumida-ku, Tokyo, 1318501 (JP); KUWAHARA, Kazuo, Minato, Wakayama-shi, Wakayama, 6408580 (JP); NAKAMURA, Sugiko, Minato, Wakayama-shi, Wakayama, 6408580 (JP)
(74) Representative: HOFFMANN EITLE
(86) International application number: PCT/JP2006/305375
(87) International publication number: WO 2006/101038

(56) References cited:
- WO-A-00/28048
- WO-A-02/30368
- WO-A2-2004/028485
- JP-A- 2001 055 310
- JP-A- 2001 507 398
- JP-A- 2003 521 489
- JP-A- 2004 149 772
- US-B1- 6 228 348

## Description

### FIELD OF THE INVENTION

The present invention relates to cosmetics for eyelashes.

### BACKGROUND OF THE INVENTION

In order to enhance make-up effects of mascara for making eyes attractive, its capability for curling eyelashes upwardly, namely the curl-up effect thereof is of a significant importance. Conventionally, to enhance the curl-up effect of mascara, there have been proposed the methods of using various film-forming agents or blending a powder therein. For example, JP 2003-95874A discloses mascara using a candelilla resin obtained by subjecting a candelilla wax having a low melting point and a high softness to fractional extraction with an organic solvent in order to attain a good curl-up effect and a good voluminous feel. Also, JP 1999-255619A discloses mascara obtained from the combination of a film-forming polymer capable of adhering to keratinous fibers and causing the stratum corneum to be contracted more than 1% and a high-hardness wax for allowing the mascara to exhibit both a curl-up effect and an effect of increasing the concentration of the color of eyelashes. However, these conventional mascara compositions still have problems such as the formation of lumps on eyelashes upon use and insufficient make-up upon finishing as well as sagging of the curled eyelashes with the passage of time, i.e., a poor curl retention effect. JP 2003-55136A discloses mascara obtained from the combination of a polymer adherable to a keratinous substance, and particles of the other polymer or waxes to impart a good curl-up effect to eyelashes. In addition, JP 2004-315420A discloses the method of enhancing a curl-up effect and a curl retention effect by using the combination of a non-aqueous polymer dispersion and a hollow powder. However, the mascara described in JP 2004-315420A also has problems such as insufficient curl retention effect and formation of lumps on eyelashes upon use. Further, if an amount of the powder blended in the mascara is increased to such an extent capable of exhibiting a good curl retention effect, a coating film obtained from the mascara tends to be deteriorated in uniformity, resulting in problems such as poor make-up upon finishing and poor retention of the make-up.

### SUMMARY OF THE INVENTION

The present invention provides a cosmetic for eyelashes which is capable of simply imparting a good curl-up effect to eyelashes, is free from formation of lumps upon coating, and can exhibit a high moisture resistance and a high curl retention effect during use as well as a beautiful finish.

The present invention relates to a cosmetic for eyelashes as defined in claim 1 which includes a film-forming polymer (A) containing constitutional units derived from methacrylic acid and/or a salt thereof (hereinafter also referred to merely as a "monomer (a)") and a monomer (b) having a solubility of 2 g or less in 100 g of water as measured at 20°C, and having a glass transition temperature (Tg₁) of 80°C or higher.

### EFFECT OF THE INVENTION

The cosmetic for eyelashes according to the present invention is capable of simply imparting a good curl-up effect to eyelashes, is free from formation of lumps upon coating, and can exhibit a high moisture resistance and a high curl retention effect during use as well as a beautiful finish.

### DETAILED DESCRIPTION OF THE INVENTION

The cosmetic for eyelashes according to the present invention includes a film-forming polymer (A) containing constitutional units derived from methacrylic acid and/or a salt thereof (monomer (a)) and a monomer (b) having a solubility of 2 g or less in 100 g of water as measured at 20°C.

### [Polymer (A)]

The polymer (A) used in the present invention is a polymer having a film- forming property which contains constitutional units derived from methacrylic acid and/or a salt thereof (monomer (a)) and a monomer (b) having a solubility of 2 g or less in 100 g of water as measured at 20°C, and has a glass transition temperature (Tg₁) of 80°C or higher. The "film-forming property" used herein means a property capable of forming a coating film when an aqueous solution or a water dispersion of the polymer is spread over a petri dish and then dried at 25°C. However, the coating film may be cracked upon the drying or may not necessarily be obtained in the form of a single sheet.

In view of enhancing the curl-up effect and moisture resistance, the polymer (A) is required to have a glass transition temperature (Tg₁) of 150°C or higher. The glass transition temperature (Tg₁) of the polymer (A) is preferably as high as possible in view of a good curl-up effect. However, the glass transition temperature (Tg₁) of the polymer (A) is 230°C or lower in view of limitations to the production thereof. When the glass transition temperature (Tg₁) of the polymer (A) is adjusted to a higher value, it is considered that the polymer (A) is enhanced in its shrinking property, resulting in a high curl-up effect.

Meanwhile, the glass transition temperature (Tg₁) is the value measured using a differential scanning colorimeter (DSC). More specifically, in the case where the DSC measurement is performed according to the following series of temperature programs (1) to (4), the glass transition temperature (Tg₁) means the value measured in the temperature program (3).

### Temperature Programs:

1. From 30 to 250°C; temperature rise rate: 30°C/min; retention time: 1 min
2. From 250 to -50°C; temperature drop (cooling) rate: 200°C/min; retention time: 10 min
3. From -50 to 250°C; temperature rise rate: 10°C/min; retention time: 1 min
4. From 250 to 30°C; temperature drop rate: 30°C/min; retention time: 2 min

The polymer (A) preferably has a weight-average molecular weight of from 5000 to 1000000 and more preferably from 8000 to 500000 as measured in terms of polystyrene by GPC. When the weight-average molecular weight of the polymer (A) is 5000 or more, the resultant cosmetic exhibits a sufficient curl-up effect, whereas when the weight-average molecular weight of the polymer (A) is 1000000 or less, the resultant cosmetic exhibits a good coating property.

The polymer (A) may be used in any configuration including an aqueous solution, a water/alcohol mixed solution, or a dispersion (emulsion) thereof.

The carboxyl groups contained in the polymer (A) may or may not be neutralized. In view of the water solubility of the polymer as well as a good curl-up effect and a good curl retention effect of the resultant cosmetic, a whole or part of the carboxyl groups contained in the polymer (A) are preferably neutralized. The degree of neutralization of the carboxyl groups contained in the polymer (A) is preferably from 0.01 to 0.6, more preferably from 0.01 to 0.5 and even more preferably from 0.02 to 0.3. The neutralizing agent used for the above neutralization may be either an inorganic base or an organic base. Examples of the inorganic base include ammonia, potassium hydroxide and sodium hydroxide. Examples of the organic base include L-arginine, 2-amino-2-methyl-1-propanol, 2-amino-1-methyl-1,3-propanediol, triethanolamine and tris(hydroxymethyl)aminomethane. The neutralizing agent may be composed of one or more basic compounds. Among these bases, in order to ensure a high curl-up effect and a high curl retention effect, preferred are inorganic bases, and more preferred is ammonia, whereas in order to obtain a good make-up persistency, preferred are organic bases, and more preferred is 2-amino-2-methyl-1-propanol.

### [Monomer (a)]

The monomer (a) used in the present invention is methacrylic acid and/or a salt thereof. Examples of the salt of methacrylic acid include ammonium methacrylate. The weight ratio of methacrylic acid to the salt thereof is not particularly limited. As the monomer (a), methacrylic acid may be used alone, or the salt of methacrylic acid may be used alone.

The content of the monomer (a) in the polymer (A) is from 20 to 90% by weight. In order to attain a sufficient curl-up effect, the content of the monomer (a) in the polymer (A) is preferably from 40 to 80% by weight.

The bases used for forming the salt of methacrylic acid may be identical to those used as the neutralizing agent for the polymer (A). Examples of the inorganic base include ammonia, potassium hydroxide and sodium hydroxide. Examples of the organic base include L-arginine, 2-amino-2-methyl-1-propanol, 2-amino-1-methyl-1,3-propanediol, triethanolamine and tris(hydroxymethyl)aminomethane. The neutralizing agent may be composed of one or more basic compounds. Among these bases, in order to ensure a high curl-up effect and a high curl retention effect, preferred are inorganic bases, and more preferred is ammonia, whereas in order to obtain a good make-up persistency, preferred are organic bases, and more preferred is 2-amino-2-methyl-1-propanol.

### [Monomer (b)]

The monomer (b) used in the present invention preferably has a solubility of 2 g or less in 100 g of water as measured at 20°C. As the solubility of the monomer (b) in water is lowered, formation of lumps upon coating tends to hardly occur, so that the resultant cosmetic for eyelashes is suitably improved in moisture resistance, i.e., can exhibit a high moisture resistance.

The homopolymer of the monomer (b) preferably has a glass transition temperature (Tg₂) of 80°C or higher and more preferably 100°C or higher in view of a good curl-up effect, and even more preferably 250°C or lower in view of limitations to production thereof.

The content of the monomer (b) in the polymer (A) is preferably from 2 to 89% by weight. In order to attain sufficient curl retention effect and moisture resistance, the content of the monomer (b) in the polymer (A) is more preferably from 2 to 80% by weight, even more preferably from 10 to 80% by weight and even more preferably from 20 to 60% by weight. Also, in order to attain a beautiful finish, the content of the monomer (b) in the polymer (A) is preferably from 40 to 95% by weight, more preferably from 50 to 90% by weight and even more preferably from 60 to 85% by weight.

Among the compounds as the monomer (b), in view of a good curl-up effect, there are used methyl methacrylate, tert-butyl methacrylate, tert-butyl acrylate, isobornyl acrylate and isobornyl methacrylate (represented by the following general formula (1)), dicyclopentanyl acrylate and dicyclopentanyl methacrylate (represented by the following general formula (2)), styrene, tert-butyl acrylamide and tert-butyl methacrylamide, and more preferred are methyl methacrylate and tert-butyl acrylate.

Meanwhile, these compounds as the monomer (b) may be used alone or in combination of any two or more thereof. 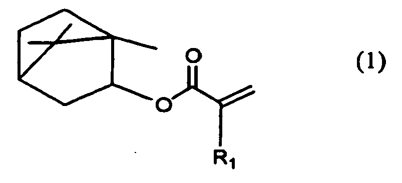 R₁ = H: Isobornyl acrylate
R₁ = CH₃: Isobornyl methacrylate 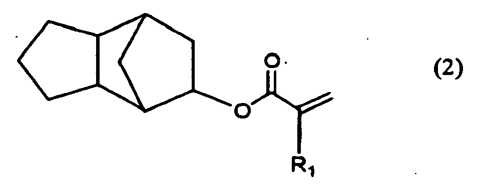 R₁ = H: Dicyclopentanyl acrylate
R₁ = CH₃: Dicyclopentanyl methacrylate

### [Cosmetic for Eyelashes]

The cosmetic for eyelashes according to the present invention contains the film-forming polymer (A). The cosmetic of the present invention preferably further contains an oil component which is kept in a solid state at 25°C and more preferably further contains a powder having an average particle size of from 1 to 20 µm.

In view of attaining a good curl-up effect as well as a beautiful finish without formation of lumps even upon repeatedly coating eyelashes with the cosmetic in a wet-on-wet manner, the content of the film-forming polymer (A) in the cosmetic is from 1 to 10% by weight and more preferably from 2 to 6% by weight.

The cosmetic for eyelashes according to the present invention preferably further contains, in addition to the above film-forming polymer (A), the oil component (wax) which is kept in a solid state at 25°C. The wax used herein may be appropriately selected from animal waxes, vegetable waxes, mineral waxes and synthetic waxes. Specific examples of the wax include carnauba wax, beeswax, extremely hydrogenated jojoba oil, lanolin wax, microcrystalline wax, paraffin wax, glycerides kept in a solid state at ordinary temperature (25°C), and silicone wax. These waxes may be used alone or in the form of a mixture of any two or more thereof.

Among these waxes, especially in order to achieve a high curl-up effect, more preferred are waxes having a rate of penetration of 8 or more. The rate of penetration used herein may be measured according to JIS K-2235-5.4. More specifically, the rate of penetration is expressed by the value which is 10 times a penetration distance (mm) obtained by measuring a length of a portion of a needle specified therein (mass of needle: 2.5±0.02 g; mass of needle holder: 47.5±0.02 g; mass of weight: 50±0.05 g) which portion is penetrated into a sample wax maintained at 25°C±0.1°C for 5 s. More specifically, among these waxes, preferred are those having a rate of penetration of 8 or more. Specific examples of the preferred wax having a rate of penetration of 8 or more include beeswax and microcrystalline wax. These waxes having a rate of penetration of 8 or more may be used alone or in the form of a mixture of any two or more thereof.

The content of the oil component (wax) which is kept in a solid state at 25°C in the cosmetic for eyelashes is preferably from 0.1 to 40% by weight, more preferably from 5 to 30% by weight and even more preferably from 10 to 25% by weight. Also, the content of the wax having a rate of penetration of 8 or more in the cosmetic for eyelashes is preferably from 0.1 to 20% by weight, more preferably from 0.5 to 10% by weight and even more preferably from 1 to 6% by weight. When the content of the oil component (wax) which is kept in a solid state at 25°C is 0.1% by weight or more, the resultant cosmetic exhibits a sufficient curl-up effect of eyelashes. On the other hand, when the content of the oil component (wax) which is kept in a solid state at 25°C is 40% by weight or less, it can be expected to achieve a beautiful appearance of eyelashes upon finishing.

The cosmetic for eyelashes according to the present invention preferably further contains a powder. The powder used in the present invention is not particularly limited as long as it has an average particle size of from 1 to 20 µm. In view of a good curl-up effect, the average particle size of the powder is preferably from 2 to 10 µm. Meanwhile, the average particle size of the powder means a median diameter measured by a laser diffraction method using a laser diffraction/light-scattering particle size distribution measuring apparatus (for example, "LA-920" available from Horiba Seisakusho Co., Ltd.).

The powder used in the present invention may have any suitable shape such as a spherical shape, a flat plate shape, a granular shape, an acicular shape, a bar shape and an amorphous shape. Among these shapes, especially preferred is a spherical shape.

The powder used in the present invention may be either an inorganic powder or an organic powder. Specific examples of the inorganic powder include plate-shaped inorganic powders such as talc, mica, sericite and kaolin, and spherical or amorphous inorganic powders such as silica, barium sulfate, aluminum hydroxide, calcium carbonate, magnesium silicate, calcium carbonate, aluminum silicate and magnesium carbonate. Specific examples of the organic powder include powders of polyamide resins, polyethylene resins, polypropylene resins, poly(methyl methacrylate) resins, cellulose-based resins, polystyrene resins, styrene-acrylic acid copolymers and silicone resins.

Among these powders, preferred are inorganic powders, more preferred are spherical inorganic powders, and even more preferred is silica powder. Meanwhile, the organic powders may be hydrophilized to render a configuration thereof more suitable.

These powders may be used alone or in the form of a mixture of any two or more thereof. In order to achieve a beautiful appearance upon finishing and attain a high curl-up effect, the powder is preferably contained in an amount of from 0.1 to 20% by weight and more preferably from 1 to 8% by weight on the basis of the total weight of the cosmetic for eyelashes.

The powder may be used in the form of a water dispersion. In this case, the powder may be dispersed in water using a water-soluble polymer or a surfactant. Specific examples of the water dispersion of the powder include a polyethylene dispersion, a silicone resin dispersion, a polystyrene resin dispersion, an urethane dispersion and a nylon dispersion.

The cosmetic for eyelashes according to the present invention may also contain a pigment. The pigment is not particularly limited as long as it can be used in ordinary cosmetic compositions. Examples of the pigment include inorganic pigments and organic pigments.

Specific examples of the inorganic pigments include titanium oxide, black titanium oxide, zinc white, red iron oxide, yellow iron oxide and black iron oxide. Specific examples of the organic pigments include tar pigments. The pigment is preferably contained in an amount of from 0.1 to 20% by weight and more preferably from 1 to 8% by weight on the basis of the total weight of the cosmetic for eyelashes.

The powder and the pigment may be subjected to surface treatments. Examples of the surface treatments include hydrophilic surface treatments such as silica treatment, alumina treatment, silica-alumina treatment and polyacrylic acid treatment, and hydrophobic surface treatments such as silicone treatment, fluorine compound treatment, metal soap treatment, lecithin treatment and oils and fats treatment.

Among them, the powders and pigments subjected to the hydrophilic treatments have a high affinity to the polymer (A). In particular, the powders and pigments treated with a polyacrylic acid are more preferred in view of a good curl-up effect and a good appearance upon finishing.

The cosmetic for eyelashes according to the present invention may further contain a water-soluble polymer as a dispersant for the powders and pigments or as a stabilizer for the dispersion system. Specific examples of the water-soluble polymer include polyvinyl alcohol, hydroxyethyl cellulose, carboxymethyl cellulose, methyl cellulose, cationized hydroxyethyl cellulose, polyvinyl pyrrolidone, polyethylene glycol, carboxyvinyl polymers, vinyl pyrrolidone/vinyl alcohol copolymers, polymethyl vinyl ether, gum arabic, guar gum, xanthan gum, modified cone starches, starches and sodium alginate. Among these water-soluble polymers, in view of a good dispersibility of the powders and pigments and a good curl-up effect, preferred are polyvinyl alcohol, hydroxyethyl cellulose, polyvinyl pyrrolidone, gum arabic and xanthan gum. The content of the water-soluble polymer in the cosmetic for eyelashes according to the present invention is preferably from 0.1 to 20% by weight and more preferably from 1 to 5% by weight.

The cosmetic for eyelashes according to the present invention may further contain a film-forming polymer emulsion constituted of a film-forming polymer. The film-forming polymer used in the polymer emulsion is selected from those polymers other than the polymer (A). When using the polymer emulsion in combination with the polymer (A), the resultant cosmetic can exhibit excellent water resistance and softness without deterioration in the shrinking property of the polymer.

Examples of the polymer emulsion include acrylic acid-based polymer emulsions, vinyl acetate-based polymer emulsions and silicone-based polymer emulsions. More specifically, among these polymer emulsions, preferred are the polymer emulsions constituted from (meth)acrylic acid or a salt thereof/(C₃ or C₄) alkyl (meth)acrylate/methyl (meth)acrylate copolymers; more preferred are the polymer emulsions constituted from (meth)acrylic acid or a salt thereof/butyl (meth)acrylate/methyl (meth)acrylate copolymers; and even more preferred are the polymer emulsions constituted from acrylic acid or a salt thereof/butyl acrylate/methyl methacrylate copolymers.

The content of the (meth)acrylic acid or salt thereof in the whole monomers constituting the above copolymers is preferably from 0.1 to 10% by weight and more preferably from 1 to 5% by weight. The content of the (C₃ or C₄) alkyl (meth)acrylate in the whole monomers constituting the above copolymers is preferably from 20 to 80% by weight and more preferably from 40 to 75% by weight. The content of the methyl (meth)acrylate in the whole monomers constituting the above copolymers is preferably from 10 to 70% by weight and more preferably from 20 to 60% by weight.

In view of a good curl-up effect and a good curl retention effect, the polymer constituting the above polymer emulsion has a glass transition temperature (Tg₃) of from -25 to 15°C and more preferably from 0 to 10°C.

Meanwhile, the glass transition temperature (Tg₃) is the value determined by measuring a glass transition temperature of a coating film obtained by drying the polymer emulsion under reduced pressure, using a differential scanning colorimeter (DSC) under the following conditions. More specifically, when performing the following series of temperature programs (1) to (4), the glass transition temperature (Tg₃) means the value measured in the temperature program (3).

### Temperature Programs:

1. From 30 to 150°C; temperature rise rate: 30°C/min; retention time: 1 min
2. From 150 to -50°C; temperature drop (cooling) rate: 200°C/min; retention time: 10 min
3. From -50 to 150°C; temperature rise rate: 10°C/min; retention time: 1 min
4. From 150 to 25°C; temperature drop rate: 30°C/min; retention time: 2 min

The above polymer emulsion is preferably produced through the polymerization process using an emulsifier such as a surfactant. As the emulsifier, there may be used those emulsifiers for ordinary polymerization processes. Among these emulsifiers, in view of attaining excellent water resistance and softness without deterioration in the shrinking property of the polymer (A), preferred are anionic surfactants, and more preferred is sodium N-stearoyl-N-methyl taurine. The amount of the emulsifier used is preferably from 0.1 to 3 parts by weight and more preferably from 0.2 to 1.5 parts by weight on the basis of 100 parts by weight of the polymer contained in the polymer emulsion.

The content of the polymer emulsion in the cosmetic for eyelashes according to the present invention is preferably from 0.1 to 40% by weight, more preferably from 1 to 30% by weight and even more preferably from 3 to 20% by weight. Also, the content of the polymer in the polymer emulsion is preferably from 20 to 60% by weight and more preferably from 30 to 55% by weight.

In addition, the weight ratio of the polymer (A) to the polymer constituting the polymer emulsion is preferably from 1/9 to 7/3 and more preferably from 2/8 to 6/4 in view of achieving both a good shrinking property of the polymer and a good softness of the resultant cosmetic.

In order to attain a good long lash effect, the cosmetic for eyelashes according to the present invention may also contain a fiber. As the fiber, there may be used any of natural fibers such as cotton, silk and hemp, regenerated fibers such as rayon, and synthetic fibers such as polyamides, polyesters, acrylic resins and polyolefins. In view of good strength, among these fibers, preferred are polyamide fibers such as nylons. If required, the fiber may be surface-treated. Examples of the surface treatment include a silica treatment, a silicone treatment, a fluorine compound treatment, a metal soap treatment and an oils-and-fats treatment.

In view of good adhesion to eyelashes, the fiber preferably has a fineness of from 0.1 to 20T and a length of from 0.1 to 5 mm. The content of the fiber in the cosmetic for eyelashes according to the present invention is preferably from 0.1 to 6% by weight on the basis of a total weight of the cosmetic in view of attaining a sufficient long lash effect.

Further, the cosmetic for eyelashes according to the present invention may also contain, as the other components, a non-volatile component which is kept in a liquid state at 25°C, a polyol and a surfactant.

Examples of the non-volatile component which is kept in a liquid state at 25°C include hydrocarbon-based oils such as liquid isoparaffins, liquid paraffins and heavy liquid isoparaffins; esters or triglycerides such as diisostearyl malate, isotridecyl isononanoate, glyceryl dimyristate, glyceryl diisostearate, glyceryl myristate isostearate, neopentyl glycol dicaprate, castor oil, macadamia nut oil and jojoba oil; and silicone oils such as dimethyl polysiloxane and methylphenyl polysiloxane. In view of attaining a beautiful finish, these non-volatile components may be contained in the cosmetic for eyelashes according to the present invention in an amount of from 0.01 to 10% by weight.

Examples of the polyols include ethylene glycol, diethylene glycol, polyethylene glycol, polypropylene glycol, propylene glycol, dipropylene glycol, 1,3-butylene glycol and glycerol. Among these polyols, in view of easiness of use, preferred are propylene glycol, dipropylene glycol and 1,3-butylene glycol, and further in view of attaining a beautiful finish, more preferred is 1,3-butylene glycol. These polyols may be contained in the cosmetic for eyelashes according to the present invention in an amount of from 0.01 to 10% by weight.

Examples of the surfactant include anionic surfactants, cationic surfactants, nonionic surfactants and amphiphatic surfactants. These surfactants may be used alone or in combination of any two or more thereof.

Further, the cosmetic for eyelashes according to the present invention may also contain a volatile oil agent. Examples of the volatile oil agent include volatile hydrocarbon oils having 8 to 16 carbon atoms and volatile ones of linear or cyclic silicones represented by the following general formula (3) or (4): 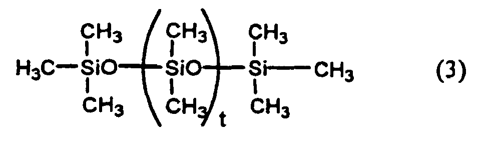 wherein t is an integer of 0 to 3, or 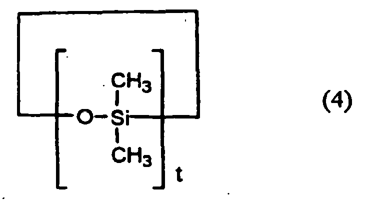 wherein u is an integer of 3 to 5.

Examples of the hydrocarbon oils having 8 to 16 carbon atoms include petroleum-derived hydrocarbon oils such as isoparaffins (light isoparaffins) and isodecane (2,2,4,4,6-pentamethyl heptane). Also, examples of the linear or cyclic silicones represented by the general formula (3) or (4) include octamethyl trisiloxane, decamethyl tetrasiloxane, octamethyl cyclotetrasiloxane and decamethyl cyclopentasiloxane.

These volatile oil agents are capable of controlling a drying velocity of the cosmetic for eyelashes according to the present invention, and may be used alone or in the form of a mixture of any two or more thereof. The amount of the volatile oil agents blended in the cosmetic for eyelashes according to the present invention is preferably from 0.5 to 20% by weight

Further, the cosmetic for eyelashes according to the present invention may also contain water and a lower alcohol having 1 to 4 carbon atoms. Examples of the lower alcohol include methanol, ethanol, isopropanol and butyl alcohol. Among these lower alcohols, in view of easiness of handling, preferred is ethanol. These lower alcohols having 1 to 4 carbon atoms may be used alone or in the form of a mixture of any two or more thereof. In view of controlling a drying velocity of a solvent for the polymers or the cosmetic for eyelashes, the content of water and the lower alcohol having 1 to 4 carbon atoms in the cosmetic for eyelashes according to the present invention is preferably from 1 to 80% by weight and more preferably from 10 to 50% by weight. In addition, the content of the lower alcohol in the cosmetic for eyelashes according to the present invention is preferably from 0.5 to 20% by weight and more preferably from 1 to 10% by weight.

Also, the cosmetic for eyelashes according to the present invention may contain, in addition to the above components, various additive components which are blended in ordinary cosmetics for imparting a good make-up effect thereto, unless the addition thereof adversely affects the effects of the present invention. Examples of the additive components include at least one of a thickening agent, an ultraviolet absorber, an ultraviolet light-scattering agent, a humectant, an antioxidant, a perfume and an antiseptic agent.

The cosmetic for eyelashes according to the present invention may be in the form of any of an oil-in-water (O/W) type emulsion composition, a water-in-oil (W/O) type emulsion composition and a composition containing only a liquid component other than water as the volatile component thereof. Among these compositions, in view of enhancing a curl-up effect, more preferred are the water-containing compositions, and most preferred are the oil-in-water (O/W) type emulsion compositions containing water or the lower alcohol having 1 to 4 carbon atoms.

The cosmetic for eyelashes according to the present invention may be produced by ordinary methods, for example, by uniformly mixing the above respective components with each other while stirring. More specifically, the cosmetic for eyelashes according to the present invention may be produced by mixing an aqueous solution or a suspension of the polymer (A), a water phase containing pigments, etc., and a wax with each other under heating at a temperature not lower than a melting point of the wax, and then cooling the resultant mixture.

The cosmetic for eyelashes according to the present invention is intended to be used as a make-up cosmetic for eyelashes, more specifically, as mascara. The cosmetic of the present invention may be used not only as the make-up cosmetic containing a coloring pigment but also as a so-called primer or top coat for eyelashes.

When the cosmetic for eyelashes according to the present invention is used as the make-up cosmetic, the cosmetic may be applied to eyelashes with a brush, etc., which may be used for ordinary mascara. In particular, a more excellent curl effect can be attained by increasing an amount of the cosmetic adhering onto the upper surface of eyelashes. For example, the cosmetic may be applied to eyelashes from above or repeatedly applied thereto from beneath using an ordinary coating tool for mascara such as a brush, a comb-shaped applicator, a coil-shaped applicator or brush, a Flocky and a bar-shaped applicator, so that the coating amount of the cosmetic applied onto the upper surface of eyelashes is increased as compared to that applied to the lower surface of eyelashes, resulting in a good curl-up effect.

### EXAMPLES

The present invention is described in more detail by referring to the following Examples and Comparative Examples.

### PRODUCTION EXAMPLE 1: Production of Aqueous Solution of Poly(Methacrylic Acid/Methyl Methacrylate) Sodium Salt (PMAA/MMA(Na))

A glass reaction vessel was charged with 2000 g of ethanol, and an interior of the reaction vessel was subjected to bubbling with nitrogen for 30 min at room temperature and then heated to 63°C under a nitrogen atmosphere. Thereafter, a solution prepared by dissolving 5.3 g of 2,2'-azobis(2,4-dimethyl valeronitrile) as an azo-based polymerization initiator ("V-65" available from Wako Junyaku Co., Ltd.) in 30 g of ethanol was added to the reaction vessel, and then a mixture composed of 230 g of methacrylic acid and 270 g of methyl methacrylate both available from Mitsubishi Gas Chemical Co., Ltd., was charged into the reaction vessel over 2 h. After completion of adding the mixture, the contents of the reaction vessel were stirred at 63°C for 6 h, and then heated to 70°C at which the respective components were polymerized while stirring for 2 h.

The resultant polymer solution was dropped into a mixed solution containing methanol and water at a blending ratio of 1:2 to subject the polymer to reprecipitation. The resultant precipitate was recovered and dried under reduced pressure at 70°C for 12 h or longer, thereby obtaining poly(methacrylic acid/methyl methacrylate). As a result of subjecting the resultant polymer to NMR measurement, it was confirmed that the weight ratio of methacrylic acid to methyl methacrylate in the polymer was 44:56, and the weight-average molecular weight of the polymer was 72000 in terms of polystyrene as measured by GPC.

Next, 80 g of the thus obtained poly(methacrylic acid/methyl methacrylate) was dissolved in 270 g of ethanol, and 86.7 g of a 1N sodium hydroxide aqueous solution was dropped into the resultant solution. After further adding 360 g of water, the reaction solution was heated by an evaporator to distil off ethanol therefrom, thereby obtaining a translucent aqueous solution of a poly(methacrylic acid/methyl methacrylate) sodium salt (degree of neutralization: 0.2). As a result, it was confirmed that the resultant polymer solution had a pH of 6 and a solid content of 20%.

In addition, as a result of the measurement using a differential scanning calorimeter "DSC 6200" available from Seiko Instruments Co., Ltd., it was confirmed that the glass transition temperature (Tg₁) of the obtained polymer was 193°C. Meanwhile, the glass transition temperature (Tg₂) of a homopolymer of methyl methacrylate was 105°C.

### PRODUCTION EXAMPLE 2: Production of Water Suspension of Poly(Methacrylic Acid/tert-Butyl Acrylamide) Sodium Salt (PMAA/tBuAAm(Na))

A glass reaction vessel was charged with 1900 g of ethanol, and an interior of the reaction vessel was subjected to bubbling with nitrogen for 30 min at room temperature and then heated to 63°C under a nitrogen atmosphere. Thereafter, a solution prepared by dissolving 6.1 g of 2,2'-azobis(2,4-dimethyl valeronitrile) as an azo-based polymerization initiator ("V-65" available from Wako Junyaku Co., Ltd.) in 30 g of ethanol was added to the reaction vessel, and then a mixture composed of 300 g of methacrylic acid, 300 g of tert-butyl acrylamide and 490 g of ethanol was charged into the reaction vessel over 2 h. After completion of adding the mixture, the contents of the reaction vessel were stirred at 63°C for 6 h, and then heated to 71°C at which the respective components were polymerized while stirring for 2 h.

The resultant polymer solution was dropped into hexane to subject the polymer to reprecipitation. The resultant precipitate was recovered and dried under reduced pressure at 70°C for 12 h or longer, thereby obtaining poly(methacrylic acid/tert-butyl acrylamide). As a result of subjecting the resultant polymer to NMR measurement, it was confirmed that the weight ratio of methacrylic acid to tert-butyl acrylamide in the polymer was 56:44, and the weight-average molecular weight of the polymer was 66000 in terms of polystyrene as measured by GPC. Next, 15 g of the thus obtained poly(methacrylic acid/tert-butyl acrylamide) was dissolved in 68 g of ethanol, and 4.08 g of a 1N sodium hydroxide aqueous solution was dropped into the resultant solution. After further adding 68 g of water, the reaction solution was heated by an evaporator to distil off ethanol therefrom, thereby obtaining a whitely turbid water suspension of a poly(methacrylic acid/tert-butyl acrylamide) sodium salt (degree of neutralization: 0.04). As a result, it was confirmed that the resultant polymer solution had a pH of 5.0 and a solid content of 32%.

In addition, as a result of the measurement using a differential scanning calorimeter "DSC 6200" available from Seiko Instruments Co., Ltd., it was confirmed that the glass transition temperature (Tg₁) of the obtained polymer was 198°C.

Meanwhile, the glass transition temperature (Tg₂) of a homopolymer of tert-butyl acrylamide was 135°C.

### PRODUCTION EXAMPLE 3: Production of Aqueous Solution of Poly(Methacrylic Acid/Methyl Methacrylate) Ammonium Salt (PMAA/MMA Ammonium Salt)

Sixty grams of the poly(methacrylic acid/methyl methacrylate) obtained in Production Example 1 was dissolved in 180 g of ethanol, and 42 mL of a 1N ammonia solution was dropped into the resultant solution. After further adding 360 g of water, the reaction solution was heated by an evaporator to distil off ethanol therefrom, thereby obtaining a translucent aqueous solution of a poly(methacrylic acid/methyl methacrylate) ammonium salt (degree of neutralization: 0.12). As a result, it was confirmed that the resultant polymer solution had a pH of 6.1 and a solid content of 20%.

In addition, as a result of the measurement using a differential scanning calorimeter "DSC 6200" available from Seiko Instruments Co., Ltd., it was confirmed that the glass transition temperature (Tg₁) of the obtained polymer was 174°C.

### PRODUCTION EXAMPLE 4: Production of Water Suspension of Poly(Methacrylic Acid/tert-Butyl Acrylamide) Ammonium Salt (PMAA/tBuAAm Ammonium Salt)

Fifteen grams of the poly(methacrylic acid/tert-butyl acrylamide) obtained in Production Example 2 was dissolved in 68 g of ethanol, and 8.3 mL of a 1N ammonia solution was dropped into the resultant solution. After further adding 270 g of water, the reaction solution was heated by an evaporator to distil off ethanol therefrom, thereby obtaining a whitely turbid water suspension of a poly(methacrylic acid/tert-butyl acrylamide) ammonium salt (degree of neutralization: 0.08). As a result, it was confirmed that the resultant water suspension of the polymer had a pH of 6.0 and a solid content of 20%.

In addition, as a result of the measurement using a differential scanning calorimeter "DSC 6200" available from Seiko Instruments Co., Ltd., it was confirmed that the glass transition temperature (Tg₁) of the obtained polymer was 197°C.

### PRODUCTION EXAMPLE 5: Production of Aqueous Solution of Poly(Methacrylic Acid/Methyl Methacrylate) 2-Amino-2-Methyl-1-Propanol Salt (PMAA/MMA(AMP))

A glass reaction vessel was charged with 793 g of ethanol, and the interior of the reaction vessel was subjected to bubbling with nitrogen for 30 min. at room temperature and then heated to 63°C under a nitrogen atmosphere. Thereafter, a solution prepared by dissolving 2.07 g of 2,2'-azobis(2,4-dimethyl valeronitrile) as an azo-based polymerization initiator ("V-65" available from Wako Junyaku Co., Ltd.) in 20 g of ethanol was added to the reaction vessel, and then a mixture composed of 54 g of methacrylic acid and 146 g of methyl methacrylate both available from Mitsubishi Gas Chemical Co., Ltd., was charged into the reaction vessel over 2 h. After completion of adding the mixture, the contents of the reaction vessel were stirred at 65°C for 4 h, and then heated to 70°C at which the respective components were polymerized while stirring for 2 h.

The resultant polymer solution was dropped into a mixed solution containing methanol and water at a blending ratio of 3:7 to subject the polymer to reprecipitation. The resultant precipitate was recovered and dried under reduced pressure at 70°C for 12 h or longer, thereby obtaining poly(methacrylic acid/methyl methacrylate). As a result of subjecting the resultant polymer to NMR measurement, it was confirmed that the weight ratio of methacrylic acid to methyl methacrylate in the polymer was 20:80, and the weight-average molecular weight of the polymer was 59700 in terms of polystyrene as measured by GPC. Also, as a result of the measurement using a differential scanning calorimeter "DSC 6200" available from Seiko Instruments Co., Ltd., it was confirmed that the glass transition temperature (Tg) of the obtained polymer was 156°C.

Next, 34.7 g of the thus obtained poly(methacrylic acid/methyl methacrylate) was dissolved in a mixed solution composed of 105 g of ethanol, 30.8 g of a 10% aqueous solution of 2-amino-2-methyl-1-propanol and 177 g of water under heating at 60°C. Thereafter, the obtained reaction solution was heated by an evaporator to distil off ethanol therefrom, thereby obtaining an aqueous solution of a poly(methacrylic acid/methyl methacrylate) 2-amino-2-methyl-1-propanol salt (degree of neutralization: 0.42). As a result, it was confirmed that the resultant polymer solution had a pH of 6.0 and a solid content of 20%.

In addition, as a result of the measurement using a differential scanning calorimeter "DSC 6200" available from Seiko Instruments Co., Ltd., it was confirmed that the glass transition temperature (Tg₁) of the obtained polymer was 172°C.

### PRODUCTION EXAMPLE 6: Production of Aqueous Solution of Poly(Methacrylic Acid/tert-Butyl Acrylamide) Sodium Salt (PMAA/tBuAAm(Na))

Fifteen grams of the poly(methacrylic acid/tert-butyl acrylamide) obtained in Production Example 2 was dissolved in 70 g of ethanol, and 8.34 g of a 1N sodium hydroxide aqueous solution was dropped into the resultant solution. After further adding 70 g of water, the reaction solution was heated by an evaporator to distil off ethanol therefrom, thereby obtaining a whitely turbid water suspension of a poly(methacrylic acid/tert-butyl acrylamide) sodium salt (degree of neutralization: 0.08). As a result, it was confirmed that the resultant water suspension of the polymer had a pH of 5.8 and a solid content of 22%.

In addition, as a result of the measurement using a differential scanning calorimeter "DSC 6200" available from Seiko Instruments Co., Ltd., it was confirmed that the glass transition temperature (Tg₁) of the obtained polymer was 213°C. Meanwhile, the glass transition temperature (Tg₂) of a homopolymer of tert-butyl acrylamide was 135°C.

### PRODUCTION EXAMPLE 7: Production of Aqueous Solution of Poly(Methacrylic Acid/tert-Butyl Methacrylate) Sodium Salt (PMAA/tBuMAA(Na))

A glass reaction vessel was charged with 394 g of ethanol, and the interior of the reaction vessel was subjected to bubbling with nitrogen for 30 min. at room temperature and then heated to 63°C under a nitrogen atmosphere. Thereafter, a solution prepared by dissolving 1.1 g of 2,2'-azobis(2,4-dimethyl valeronitrile) as an azo-based polymerization initiator ("V-65" available from Wako Junyaku Co., Ltd.) in 20 g of ethanol was added to the reaction vessel, and then a mixture composed of 50 g of methacrylic acid and 50 g of tert-butyl methacrylate both available from Mitsubishi Gas Chemical Co., Ltd., was charged into the reaction vessel over 2 h. After completion of adding the mixture, the contents of the reaction vessel were stirred at 63°C for 6 h, and then heated to 70°C at which the respective components were polymerized while stirring for 2 h.

The resultant polymer solution was dropped into hexane to subject the polymer to reprecipitation. The resultant precipitate was recovered and dried under reduced pressure at 70°C for 12 h or longer, thereby obtaining poly(methacrylic acid/tert-butyl methacrylate). As a result of subjecting the resultant polymer to NMR measurement, it was confirmed that the weight ratio of methacrylic acid to tert-butyl methacrylate in the polymer was 53:47, and the weight average molecular weight of the polymer was 55000 in terms of polystyrene as measured by GPC.

Next, 50 g of the thus obtained poly(methacrylic acid/tert-butyl methacrylate) was dissolved in 225 g of ethanol, and 76.8 g of a 1N sodium hydroxide aqueous solution was dropped into the resultant solution. After further adding 148 g of water, the reaction solution was heated by an evaporator to distil off ethanol therefrom, thereby obtaining a translucent aqueous solution of a poly(methacrylic acid/tert-butyl methacrylate) sodium salt (degree of neutralization: 0.25). As a result, it was confirmed that the resultant polymer solution had a pH of 7.0 and a solid content of 19%.

In addition, as a result of the measurement using a differential scanning calorimeter "DSC 6200" available from Seiko Instruments Co., Ltd., it was confirmed that the glass transition temperature (Tg₁) of the obtained polymer was 159°C. Meanwhile, the glass transition temperature (Tg₂) of a homopolymer of tert-butyl methacrylate was 105°C.

### PRODUCTION EXAMPLE 8: Production of Aqueous Solution of Poly(Methacrylic Acid/Isobornyl Methacrylate) Sodium Salt (PMAA/iBoMA(Na))

A glass reaction vessel was charged with 394 g of ethanol, and the interior of the reaction vessel was subjected to bubbling with nitrogen for 30 min. at room temperature and then heated to 63°C under a nitrogen atmosphere. Thereafter, a solution prepared by dissolving 1.0 g of 2,2'-azobis(2,4-dimethyl valeronitrile) as an azo-based polymerization initiator ("V-65" available from Wako Junyaku Co., Ltd.) in 20 g of ethanol was added to the reaction vessel, and then a mixture composed of 70 g of methacrylic acid and 30 g of isobornyl methacrylate both available from Mitsubishi Gas Chemical Co., Ltd., was charged into the reaction vessel over 2 h. After completion of adding the mixture, the contents of the reaction vessel were stirred at 63°C for 6 h, and then heated to 72°C at which the respective components were polymerized while stirring for 2 h.

The resultant polymer solution was dropped into hexane to subject the polymer to reprecipitation. The resultant precipitate was recovered and dried under reduced pressure at 70°C for 12 h or longer, thereby obtaining poly(methacrylic acid/isobornyl methacrylate). As a result of subjecting the resultant polymer to NMR measurement, it was confirmed that the weight ratio of methacrylic acid to isobornyl methacrylate in the polymer was 59:41, and the weight-average molecular weight of the polymer was 66000 in terms of polystyrene as measured by GPC.

Next, 50 g of the thus obtained poly(methacrylic acid/isobornyl methacrylate) was dissolved in 225 g of ethanol, and 128.7 g of a 1N sodium hydroxide aqueous solution was dropped into the resultant solution. After further adding 96 g of water, the reaction solution was heated by an evaporator to distil off ethanol therefrom, thereby obtaining a translucent aqueous solution of a poly(methacrylic acid/isobornyl methacrylate) sodium salt (degree of neutralization: 0.4). As a result, it was confirmed that the resultant polymer solution had a pH of 5.6 and a solid content of 20%.

In addition, as a result of the measurement using a differential scanning calorimeter "DSC 6200" available from Seiko Instruments Co., Ltd., it was confirmed that the glass transition temperature (Tg₁) of the obtained polymer was 217°C. Meanwhile, the glass transition temperature (Tg₂) of a homopolymer of isobornyl methacrylate was 180°C.

### PRODUCTION EXAMPLE 9: Production of Aqueous Solution of Poly(Methacrylic Acid/Dicyclopentanyl Methacrylate) Sodium Salt (PMAA/DCPMA(Na))

A glass reaction vessel was charged with 394 g of ethanol, and the interior of the reaction vessel was subjected to bubbling with nitrogen for 30 min. at room temperature and then heated to 63°C under a nitrogen atmosphere. Thereafter, a solution prepared by dissolving 0.8 g of 2,2'-azobis(2,4-dimethyl valeronitrile) as an azo-based polymerization initiator ("V-65" available from Wako Junyaku Co., Ltd.) in 20 g of ethanol was added to the reaction vessel, and then a mixture composed of 70 g of methacrylic acid and 30 g of dicyclopentanyl methacrylate both available from Mitsubishi Gas Chemical Co., Ltd., was charged into the reaction vessel over 2 h. After completion of adding the mixture, the contents of the reaction vessel were stirred at 63°C for 6 h, and then heated to 68°C at which the respective components were polymerized while stirring for 2 h.

The resultant polymer solution was dropped into hexane to subject the polymer to reprecipitation. The resultant precipitate was recovered and dried under reduced pressure at 70°C for 12 h or longer, thereby obtaining poly(methacrylic acid/dicyclopentanyl methacrylate). As a result of subjecting the resultant polymer to NMR measurement, it was confirmed that the weight ratio of methacrylic acid to dicyclopentanyl methacrylate in the polymer was 57:43, and the weight-average molecular weight of the polymer was 68000 in terms of polystyrene as measured by GPC.

Next, 50 g of the thus obtained poly(methacrylic acid/dicyclopentanyl methacrylate) was dissolved in 56 g of ethanol, and 256 g of a 1N sodium hydroxide aqueous solution was dropped into the resultant solution. After further adding 225 g of water, the reaction solution was heated by an evaporator to distil off ethanol therefrom, thereby obtaining a translucent aqueous solution of a poly(methacrylic acid/dicyclopentanyl methacrylate) sodium salt (degree of neutralization: 0.6). As a result, it was confirmed that the resultant polymer solution had a pH of 6.5 and a solid content of 20%.

In addition, as a result of the measurement using a differential scanning calorimeter "DSC 6200" available from Seiko Instruments Co., Ltd., it was confirmed that the glass transition temperature (Tg₁) of the obtained polymer was 208°C. Meanwhile, the glass transition temperature (Tg₂) of a homopolymer of dicyclopentanyl methacrylate was 175°C.

### PRODUCTION EXAMPLE 10: Production of Aqueous Solution of Poly(Methacrylic Acid/Styrene) Sodium Salt (PMAA/ST(Na))

A glass reaction vessel was charged with 394 g of ethanol, and an interior of the reaction vessel was subjected to bubbling with nitrogen for 30 min at room temperature and then heated to 63°C under a nitrogen atmosphere. Thereafter, a solution prepared by dissolving 1.1 g of 2,2'-azobis(2,4-dimethyl valeronitrile) as an azo-based polymerization initiator ("V-65" available from Wako Junyaku Co., Ltd.) in 20 g of ethanol was added to the reaction vessel, and then a mixture composed of 50 g of methacrylic acid and 50 g of styrene both available from Mitsubishi Gas Chemical Co., Ltd., was charged into the reaction vessel over 2 h. After completion of adding the mixture, the contents of the reaction vessel were stirred at 63°C for 6 h, and then heated to 70°C at which the respective components were polymerized while stirring for 2 h.

The resultant polymer solution was dropped into hexane to subject the polymer to reprecipitation. The resultant precipitate was recovered and dried under reduced pressure at 70°C for 12 h or longer, thereby obtaining poly(methacrylic acid/styrene). As a result of subjecting the resultant polymer to NMR measurement, it was confirmed that the weight ratio of methacrylic acid to styrene in the polymer was 42:58, and the weight-average molecular weight of the polymer was 37000 in terms of polystyrene as measured by GPC.

Next, 27 g of the thus obtained poly(methacrylic acid/styrene) was dissolved in 123 g of ethanol, and 77.3 g of a 1N sodium hydroxide aqueous solution was dropped into the resultant solution. After further adding 46 g of water, the reaction solution was heated by an evaporator to distil off ethanol therefrom, thereby obtaining a translucent aqueous solution of a poly(methacrylic acid/styrene) sodium salt (degree of neutralization: 0.58). As a result, it was confirmed that the resultant polymer solution had a pH of 7.4 and a solid content of 18%.

In addition, as a result of the measurement using a differential scanning calorimeter "DSC 6200" available from Seiko Instruments Co., Ltd., it was confirmed that the glass transition temperature (Tg₁) of the obtained polymer was 155°C. Meanwhile, the glass transition temperature (Tg₂) of a homopolymer of the styrene was 100°C.

### PRODUCTION EXAMPLE 11: Production of Aqueous Solution of Poly(Methacrylic Acid) Sodium Salt (PMAA(Na))

A glass reaction vessel was charged with 600 g of methacrylic acid available from Wako Junyaku Co., Ltd., 3 L of ethanol and 3.46 g of 2,2'-azobis(2,4-dimethyl valeronitrile) as an azo-based polymerization initiator ("V-65" available from Wako Junyaku Co., Ltd.), and then the contents of the reaction vessel were polymerized at 65°C for 4 h.

The resultant polymer solution was dropped into 40 L of hexane to subject the polymer to reprecipitation. The resultant precipitate was recovered and dried under reduced pressure at 65°C for 12 h or longer.

A glass reaction vessel was charged with 300 g of the thus obtained poly(methacrylic acid) and then with 975 g of water. The contents of the reaction vessel were stirred at 70°C for 12 h to dissolve the polymer in water, followed by adding 150 g of a 30% sodium hydroxide aqueous solution to the resultant solution. Thereafter, 150 g of ethanol was added to the obtained reaction solution for an antiseptic purpose thereof. As a result, it was confirmed that the resultant PMAA(Na) aqueous solution had a degree of neutralization of 0.3, a pH of 6.3 and a solid content of 22.6%, and the weight-average molecular weight of the obtained PMAA was 130000 in terms of polyethylene glycol as measured by GPC.

In addition, as a result of the measurement using a differential scanning calorimeter "DSC 6200" available from Seiko Instruments Co., Ltd., it was confirmed that the glass transition temperature (Tg₁) of the obtained polymer was 208°C.

### PRODUCTION EXAMPLE 12: Production of Polymer Emulsion

A glass reaction vessel was charged with 0.6 g of sodium N-stearoyl-N-methyl taurine "NIKKOL SMT" available from Nikko Chemical Co., Ltd., and 875 g of ion-exchanged water, and then purged with nitrogen while stirring, followed by raising an inside temperature of the reaction vessel to 70°C by heating. Next, after adding a solution prepared by dissolving 2.5 g of ammonium persulfate in 25 g of ion-exchanged water to the reaction vessel, a solution prepared by mixing 200 g of methyl methacrylate, 285 g of butyl acrylate and 15 g of acrylic acid with each other was dropped into the reaction vessel over 3 h. After completion of the dropping, the contents of the reaction vessel were reacted with each other at 70°C for 1 h, heated to 75°C at which the reaction was further conducted for 3 h. Thereafter, the reaction solution was cooled and then mixed with 73 g of a 1N sodium hydroxide aqueous solution. The obtained reaction solution was concentrated using an evaporator, thereby obtaining a polymer latex. As a result of the measurement using a particle size distribution measuring apparatus "LA-920" available from Horiba Seisakusho Co., Ltd., it was confirmed that the resultant polymer latex had a particle size of 0.1 µm. Further, it was confirmed that the polymer latex had a pH of 6.5 and a solid content of 50%.

In addition, as a result of the measurement using a differential scanning calorimeter "DSC 6200" available from Seiko Instruments Co., Ltd., it was confirmed that the glass transition temperature (Tg₃) of the polymer forming the polymer emulsion was 5°C.

### (1) Method for Evaluation of Curl-up: Hair Fiber Test

Three hair fibers each having a length of from 1 to 1.5 cm were sampled and fixed in a horizontal plane. Then, the respective mascara compositions shown in Tables 1 and 2 were applied at 23°C and a relative humidity of 60% onto the hair fibers with a brush 10 times to measure an angle of warpage of the hair fibers from the horizontal plane. The measurement was conducted three times, and an average value of the three measured values was calculated. The curl-up of the hair fibers was evaluated from the average value according to the following evaluation criteria.

### (2) Method for Evaluation of Moisture Resistance

The angle of warpage of the hair fibers was measured immediately after applying the mascara compositions thereonto at 23°C and a relative humidity of 60% according to the above method and after the thus mascara-applied hair fibers were allowed to stand at 40°C and a relative humidity of 75% for 5 min to calculate a curl retention rate represented by the following formula, as an index of the moisture resistance:

Curl Retention Rate = [(angle of warpage of hair fibers after the applied hair fiber's were allowed to stand at 40°C and a relative humidity of 75% for 5 min)/(angle of warpage of hair fibers immediately after applying the mascara compositions thereto)] x 100

### (3) Evaluation of Curl Retention Effect

The angle of warpage of the hair fibers was measured immediately after applying the mascara compositions thereonto according to the above method and after the elapse of 3 h from the time of applying the mascara compositions thereonto to calculate a curl retention rate represented by the following formula, as an index of the curl retention effect:

Curl Retention Rate = [(angle of warpage of hair fibers after the elapse of 3 h from the time of applying the mascara compositions thereto)/(angle of warpage of hair fibers immediately after applying the mascara compositions thereto)] x 100

### (4) Evaluation of Appearance upon Finishing

The hair fibers coated with the mascara compositions according to the above method were observed by the naked eyes by expert panelists to examine formation of lumps thereon and evaluate the appearance upon finishing according to the following evaluation criteria.
ⓞ: No formation of lumps
○: Slight formation of lumps but no significant influence on appearance upon finishing
Δ: Slight formation of lumps and some influence on appearance upon finishing
× : Significant formation of lumps

### (5) Evaluation of Persistency of Make-up

The respective mascara compositions prepared were applied onto eyelashes with a brush, and after the elapse of 8 h from the time of applying the mascara compositions thereto, the persistency of make-up was evaluated by 10 female panelists.
ⓞ: Among the 10 panelists, 9 or 10 persons had a good evaluation for make-up persistency;
○ Among the 10 panelists, 6 to 8 persons had a good evaluation for make-up persistency;
Δ: Among the 10 panelists, 3 to 5 persons had a good evaluation for make-up persistency;
× Among the 10 panelists, only 2 or less persons had a good evaluation for make-up persistency.

The average particle size of the powder was determined as a median diameter measured by a laser diffraction method using a laser diffraction particle size distribution measuring apparatus "LA-920" available from Horiba Seisakusho Co., Ltd.

### EXAMPLES 1 TO 3 AND COMPARATIVE EXAMPLES 1 TO 4

The respective cosmetics as a primer for eyelash having the compositions shown in Table 1 were prepared. The thus prepared cosmetics of Examples 1 to 3 and Comparative Examples 1 to 4 were used as a primer for eyelash to evaluate the curl-up effect (hair fiber test), the moisture resistance and the appearance upon finishing by the above methods (1), (2) and (4). The results are shown in Table 1.

As a result, it was confirmed that the primers for eyelash obtained in Examples 1 to 3 according to the present invention had a high curl-up effect, were excellent in moisture resistance and exhibited a sufficient appearance upon finishing. On the other hand, the primer for eyelash obtained in Comparative Example 1 had a high curl-up effect, but exhibited an extremely low moisture resistance and a poor appearance upon finishing. The primers for eyelash obtained in Comparative Examples 2 to 4 were insufficient in curl-up effect and appearance upon finishing and, therefore, unsuitable as a primer for eyelash.

**TABLE 1-1**

| | Examples | | |
|---|---|---|---|
| | 1 | 2 | 3 |
| Type of polymer (A) | PMAA/ MMA(Na) | PMAA/ tBuAAm(Na) | PMAA/ tBuAAm(Na) |
| Method for production of polymer | Production | Production | Production |
| (A) | Example 1 | Example 6 | Example 2 |
| Degree of neutralization | 0.2 | 0.08 | 0.04 |
| Tg of polymer (A) (°C) | 193 | 213 | 198 |
| Tg of homopolymer of monomer (b) (°C) | 105 | 135 | 105 |
| Composition of cosmetic (wt%) | | | |
| Polymer of Production Example 1 | 20 | - | - |
| Polymer of Production Example 6 | - | 20 | - |
| Polymer of Production Example 2 | - | - | 20 |
| Antiseptic agent | 3 | 3 | 3 |
| Purified water | Balance | Balance | Balance |
| Total | 100 | 100 | 100 |
| Evaluation items | | | |
| Curl-up effect (hair fiber) (°) | 47 | 45 | 45 |
| Moisture resistance (%) | 55 | 53 | 55 |
| Appearance upon finishing | ⓞ | ⓞ | ⓞ |

**TABLE 1-2**

| | Comparative Examples | | | |
|---|---|---|---|---|
| | 1 | 2 | 3 | 4 |
| Type of polymer | PMAA (Na) | PAA | PDMAPAAm | AQ55S |
| Method for production of polymer | Production Example 11 | *1 | *2 | *3 |
| Degree of neutralization | 0.3 | 0 | - | - |
| Tg of polymer (°C) | 160 | 106 | 36 | 55 |
| Composition of cosmetic (wt%) | | | | |
| Polymer of Production Example 12 | 20 | - | - | - |
| 25% Aqueous solution of poly(acrylic acid) | - | 20 | - | - |
| N-(3-dimethylaminopropyl) acrylamide | - | - | 20 | - |
| AQ™55S | - | - | - | 20 |
| Antiseptic agent | 3 | 3 | 3 | 3 |
| Purified water | Balance | Balance | Balance | Balance |
| Total | 100 | 100 | 100 | 100 |
| Evaluation items | | | | |
| Curl-up effect (hair fiber) (°) | 90 | 0 | 0 | 5 |
| Moisture resistance (%) | 0 | - | - | 0 |
| Appearance upon finishing | Δ | × | × | Δ |

| | | | | |
|---|---|---|---|---|
| Note: (*1): 25% Aqueous solution of poly(acrylic acid) (PAA) available from Wako Junyaku Kogyo Co., Ltd.; viscosity: 8000 to 12000 cP (*2): N-(3-dimethylaminopropyl)acrylamide (PDMAPAAm) (*3): Diglycol/cyclohexane dimethanol/isophthalate/sulfone isophthalate polymer; "AQ™55S" available from Eastman Chemical Company | | | | |

### EXAMPLES 4 TO 11 AND COMPARATIVE EXAMPLES 5 TO 7

The respective components shown in Table 2 were uniformly mixed with each other while stirring at a compositional ratio shown in Table 2, thereby preparing the mascara compositions of Examples 4 to 11 and Comparative Examples 5 to 7.

The mascara compositions thus obtained in Examples 4 to 11 and Comparative Examples 5 to 7 were evaluated with respect to the curl-up effect, the curl retention effect, the appearance upon finishing and the make-up persistency by the above methods (1), (3) to (5). The results are shown in Table 2

As a result, it was confirmed that the mascara compositions obtained in Examples 4 to 11 according to the present invention exhibited sufficient curl-up effect and curl retention effect and an excellent make-up persistency as well as a beautiful finish. On the other hand, it was confirmed that the mascara composition obtained in Comparative Example 5 using no film-forming polymer and the mascara compositions obtained in Comparative Example 6 to which no powder was added, exhibited neither curl -up effect nor curl retention effect although they exhibited the substantially same make-up persistency and appearance upon finishing as those of Examples 4 to 10. In addition, the mascara composition obtained in Comparative Example 7 to which no wax was added, was insufficient in all of curl-up effect, curl retention effect and appearance upon finishing.

**TABLE 2-1**

| | Examples | | | | |
|---|---|---|---|---|---|
| | 4 | 5 | 6 | 7 | 8 |
| (1) Polymer (A) (mass %) | | | | | |
| PMAA/MMA(Na)salt*¹ | 5 | 5 | 5 | - | - |
| PMAA/tBuAAm(Na)salt*² | - | - | - | 5 | 5 |
| PMAA/MMA ammonium salt*³ | - | - | - | - | - |
| PMAA/tBuAAm ammonium salt*⁴ | - | - | - | - | - |
| PMAA/MMA(AMP)salt*¹⁷ | - | - | - | - | - |
| (2) Wax (mass %) | | | | | |
| Carnauba wax*⁵ | 3 | 3 | 3 | 3 | 3 |
| Candelilla wax | - | - | - | - | - |
| Beeswax*⁶ | - | 7 | 7 | 7 | 7 |
| Extremely hydrogenated jojoba oil*⁷ | 6 | 6 | 6 | 6 | 6 |
| Microcrystalline wax*⁸ | - | 3 | 3 | 3 | 3 |
| Paraffin wax*⁹ | 10 | 4 | 4 | 4 | 4 |
| (3) Powder (mass %) | | | | | |
| Silica*¹⁰ | 3 | 3 | 3 | 3 | 3 |
| Talc*¹¹ | 1 | 1 | 1 | 1 | 1 |
| (4) Others (mass %) | | | | | |
| Polyvinyl alcohol*¹² | 2 | 2 | 2 | 2 | 2 |
| Polymer emulsion*¹³ | 10 | 10 | 10 | 10 | 10 |
| Polymer emulsion*¹⁸ | - | - | - | - | - |
| 1,3-Butylene glycol | 5 | - | - | 5 | 5 |
| Propylene glycol | - | 5 | 5 | - | - |
| Stearic acid | 3 | 3 | 3 | 3 | 3 |
| Polyoxyethylene glycerol monostearate*14 | 0.5 | 0.5 | 0.5 | 0.5 | - |
| Antiseptic agent (methyl parabene) | 0.2 | 0.2 | 0.2 | 0.2 | 0.2 |
| Black iron oxide | 5 | 5 | 5 | 5 | - |
| Poly(acrylic acid) treated black iron oxide*15 | - | - | - | - | 5 |
| Nylon fiber*16 | 1 | 1 | 1 | 1 | 1 |
| 2-Amino-2-methyl-1-propanol (AMP) | 0.5 | 0.5 | 0.5 | 0.5 | 0.5 |
| (5) Water and lower alcohol (mass %) | | | | | |
| Ethanol | - | - | 5 | 5 | 5 |
| Purified water | Balance | Balance | Balance | Balance | Balance |
| Total | 100 | 100 | 100 | 100 | 100 |
| Curl-up effect | 16° | 20° | 22° | 25° | 30° |
| Curl retention effect | 65% | 67% | 72% | 75% | 77% |
| Appearance upon finishing | ○ | ○ | ⓞ | ⓞ | ⓞ |
| Persistency of make-up | ○ | ○ | ○ | ○ | ○ |

**TABLE 2-2**

| | Examples | | | Comparative Examples | | |
|---|---|---|---|---|---|---|
| | 9 | 10 | 11 | 5 | 6 | 7 |
| (1) Polymer (A) (mass %) | | | | | | |
| PMAA/MMA(Na)salt*¹ | - | - | - | - | 5 | 5 |
| PMAA/tBuAAm(Na)Salt*² | - | - | - | - | - | - |
| PMAA/MMA ammonium Salt*³ | 5 | - | - | - | - | - |
| PMAA/tBuAAm ammonium salt*⁴ | - | 5 | - | - | - | - |
| PMAA/MMA(AMP)salt*¹⁷ | - | - | 3 | - | - | - |
| (2) Wax (mass %) | | | | | | |
| Carnauba wax*⁵ | 3 | 3 | - | 3 | 3 | - |
| Candelilla wax | - | - | 3 | - | - | - |
| Beeswax*⁶ | 7 | 7 | 2 | 7 | 7 | - |
| Extremely hydrogenated jojoba oil*⁷ | 6 | 6 | 3 | 6 | 6 | - |
| Microcrystalline wax*⁸ | 3 | 3 | - | 3 | 3 | - |
| Paraffin wax*⁹ | 4 | 4 | 8 | 4 | 4 | - |
| (3) Powder (mass %) | | | | | | |
| Silica*¹⁰ | 3 | 3 | 3 | 3 | - | 3 |
| Talc*¹¹ | 1 | 1 | 1 | 1 | - | 1 |
| (4) Others (mass %) | | | | | | |
| Polyvinyl alcohol*¹² | 2 | 2 | 2 | 2 | - | 2 |
| Polymer emulsion*¹³ | 10 | 10 | - | 10 | 10 | 30 |
| Polymer emulsion*¹⁸ | - | - | 10 | - | - | - |
| 1,3-Butylene glycol | - | 5 | 5 | 5 | 5 | 5 |
| Propylene glycol | 5 | - | - | - | - | - |
| Stearic acid | 3 | 3 | 3 | 3 | 3 | - |
| Polyoxyethylene glycerol monostearate*¹⁴ | 0.5 | - | - | 0.5 | 0.5 | 0.5 |
| Antiseptic agent (methyl parabene) | 0.2 | 0.2 | 0.2 | 0.2 | 0.2 | 0.2 |
| Black iron oxide | - | - | - | 5 | 5 | 5 |
| Poly(acrylic acid)-treated black iron oxide*¹⁵ | 5 | 5 | 5 | - | - | - |
| Nylon fiber*¹⁶ | 1 | 1 | 1 | 1 | 1 | 1 |
| 2-Amino-2-methyl-1-propano 1 (AMP) | 0.5 | 0.5 | 0.5 | 0.5 | 0.5 | - |
| (5) Water and lower alcohol (mass %) | | | | | | |
| Ethanol | 5 | 5 | 5 | 5 | 5 | 5 |
| Purified water | Balance | Balance | Balance | Balance | Balance | Balance |
| Total | 100 | 100 | 100 | 100 | 100 | 100 |
| Curl-up effect | 27° | 35° | 40° | 8° | 14° | 10° |
| Curl retention effect | 80% | 82% | 80% | 35% | 55% | 58% |
| Appearance upon finishing | ⓞ | ⓞ | ⓞ | ○ | ○ | × |
| Persistency of make-up | ○ | ○ | ⓞ | ○ | ○ | ○ |

| | | | | | | |
|---|---|---|---|---|---|---|
| Note: (*1) Aqueous solution of a poly(methacrylic acid/methyl methacrylate) sodium salt produced in Production Example 1 (*2) Water suspension of a poly(methacrylic acid/tert-butyl acrylamide) sodium salt produced in Production Example 2 (*3) Aqueous solution of a poly(methacrylic acid/methyl methacrylate) ammonium salt produced in Production Example 3 (*4) Water suspension of a poly(methacrylic acid/tert-butyl acrylamide) ammonium salt produced in Production Example 4 (*5) Carnauba wax: "Purified Carnauba Wax No. 1" available from Cerarica Yoda Co., Ltd. (rate of penetration: 1 or less) (*6) Beeswax: "BEES WAX-S" available from Croda Japan Co., Ltd. (rate of penetration: 18) (*7) Extremely hydrogenated jojoba oil available from Koei Kogyo Co., Ltd. (rate of penetration: 1) (*8) Microcrystalline wax: "MULTI-WAX W-445" available from Witco Corp. (rate of penetration: 34) (*9) Paraffin wax: "HNP-9" available from Nippon Seiroh Co., Ltd. (rate of penetration: 7) (*10) Silica: "SUNSFAIR L31" available from Asahi Glass Co., Ltd. (average particle size: 3 µm; spherical powder) (*11) Talc: "TALC JA-46R" available from Asada Seifun Co., Ltd. (average particle size: 8 µm; plate-shaped powder) (*12) Polyvinyl alcohol: "GOSENOL EG-30" available from Japan Synthetic Chemical Co, Ltd. (*13) Polymer emulsion: "YODOZOLE GH34" available from Nippon NSC Co., Ltd. (*14) Polyoxyethylene glycerol monostearate "NIKKOLE TMGS-15" available from Nikko Chemical Co., Ltd. (*15) Poly(acrylic acid)-treated black iron oxide: "PA-BLACK BL-100" available from Miyoshi Kasei Co., Ltd.; 3% poly(acrylic acid)-treated black iron oxide (*16) Nylon fiber (length: 2 mm; fineness: 6.7T) available from UNITIKA Co., Ltd. (*17) Aqueous solution of a poly(methacrylic acid/methyl methacrylate) 2-amino-2-methyl-1-propanol salt produced in Production Example 5 (*18) Polymer emulsion produced in Production Example 12 | | | | | | |

### EXAMPLES 12 TO 20

In Examples 12 to 20, the mascara compositions containing the following components were prepared.

### EXAMPLE 12 (% by weight)

| | | |
|---|---|---|
| Polymer produced in Production Example 5 | 6 | |
| Candelilla wax | 3 | |
| Beeswax | | 6 |
| Extremely hydrogenated jojoba oil | 3 | |
| Paraffin wax | | 8 |
| Silica | | 1 |
| Polyvinyl alcohol | | 2 |
| Polymer emulsion (Production Example 12) | 10 | |
| 1,3-Butylene glycol | | 5 |
| Stearic acid | | 3 |
| Methyl parabene | | 0.2 |
| Poly(acrylic acid)-treated black iron oxide | 5 | |
| Nylon fiber | | 1 |
| 2-Amino-2-methyl-1-propanol | 0.5 | |
| Ethanol | | 5 |
| Purified water | Balance | |
| Total | | |
| 100 | | |

### EXAMPLE 13 (% by weight)

| | | |
|---|---|---|
| Polymer produced in Production Example 5 | 2 | |
| Candelilla wax | 3 | |
| Beeswax | | 1 |
| Extremely hydrogenated jojoba oil | 3 | |
| Paraffin wax | | 8 |
| Silica | | 7 |
| Talc | | 1 |
| Polyvinyl alcohol | | 2 |
| Polymer emulsion (Production Example 12) | 10 | |
| 1,3-Butylene glycol | | 5 |
| Stearic acid | | 3 |
| Methyl parabene | | 0.2 |
| Poly(acrylic acid)-treated black iron oxide | 5 | |
| Nylon fiber | | 1 |
| 2-Amino-2-methyl-1-propanol | 0.5 | |
| Ethanol | | 5 |
| Purified water | Balance | |
| Total | | |
| 100 | | |

### EXAMPLE 14 (% by weight)

| | | |
|---|---|---|
| Polymer produced in Production Example 1 | 5 | |
| Carnauba wax^{*4} | | 15 |
| Beeswax^{*5} | | 15 |
| Stearic acid^{*6} | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

### EXAMPLE 15 (% by weight)

| | | |
|---|---|---|
| Polymer produced in Production Example 6 | 5 | |
| Carnauba wax^{*4} | | 15 |
| Beeswax*⁵ | | 15 |
| Stearic acid*⁶ | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

### EXAMPLE 16 (% by weight)

| | | |
|---|---|---|
| Polymer produced in Production Example 2 | 5 | |
| Carnauba wax*⁴ : | | 15 |
| Beeswax^{*5} | | 15 |
| Stearic acid^{*6} | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

### EXAMPLE 17 (% by weight)

| | | |
|---|---|---|
| Polymer produced in Production Example 7 | 5 | |
| Carnauba wax*⁴ | | 15 |
| Beeswax*⁵ | | 15 |
| Stearic acid*⁶ | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

### EXAMPLE 18 (% by weight)

| | | |
|---|---|---|
| Polymer produced in Production Example 8 | 5 | |
| Carnauba wax^{*4} | | 15 |
| Beeswax^{*5} | | 15 |
| Stearic acid^{*6} | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

### EXAMPLE 19 (% by weight)

| | | |
|---|---|---|
| Polymer produced in Production Example 9 | 5 | |
| Carnauba wax^{*4} | | 15 |
| Beeswax^{*5} | | 15 |
| Stearic acid^{*6} | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

### EXAMPLE 20 (% by weight)

| | | |
|---|---|---|
| Polymer produced in Production Example 10 | 5 | |
| Carnauba wax^{*4} | | 15 |
| Beeswax^{*5} | | 15 |
| Stearic acid^{*6} | 2 | |
| 2-Amino-2-methyl-1-propanol | 1 | |
| Polyoxyethylene (EO = 15) glycerol monostearate | 0.5 | |
| Black iron oxide | | 10 |
| Antiseptic agent | | 0.2 |
| Purified water | Balance | |
| Total | | |
| 100 | | |

| | | |
|---|---|---|
| Note: (*4) Carnauba wax: "Purified Carnauba Wax No. 1" available from Cerarica Yoda Co., Ltd. (rate of penetration: 1 or less); (*5) Beeswax: "BEES WAX-S" available from Croda Japan Co., Ltd. (rate of penetration: 18); (*6) Stearic acid: "Purified Stearic Acid 700V" available from Kao Corp. | | |

The mascara compositions of Examples 12 to 20 according to the present invention all exhibited a sufficient curl-up effect, an excellent moisture resistance and a good appearance upon finishing.

### INDUSTRIAL APPLICABILITY

The cosmetic for eyelashes according to the present invention can be suitably used as a make-up cosmetic for eyelashes, more specifically as a mascara, and can simply impart a good curl-up effect to eyelashes, is free from formation of lumps upon coating, and further exhibits a high moisture resistance, a high curl retention effect during use and a beautiful finish.

## Claims

1. A cosmetic for eyelashes comprising 1 to 10 % by weight, based on the total weight of the cosmetic, of a film-forming polymer (A) containing 20 to 90 by weight of a monomer (a), wherein the monomer (a) are constitutional units derived from methacrylic acid and/or a salt thereof and a monomer (b) having a solubility of 2 g or less in 100 g of water as measured at 20°C, and having a glass transition temperature (Tg₁) of from 150 to 230°C,
wherein the monomer (b) is selected from at least one monomer or more selected from the group consisting of methyl methacrylate, tert-butyl methacrylate, tert-butyl acrylate, isobornyl acrylate, isobornyl methacrylate, represented by the general formula (1), dicyclopentanyl acrylate, dicyclopentanyl methacrylate, represented by the general formula (2), styrene, tert-butyl acrylamide and tert-butyl methacrylamide 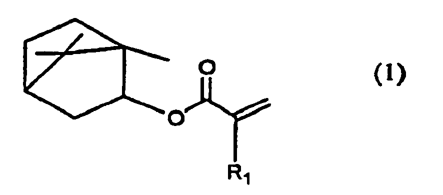 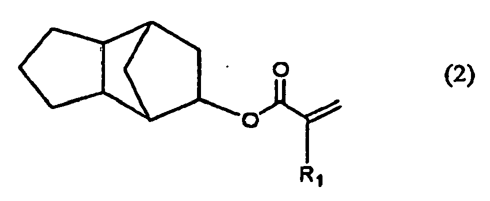 wherein R₁ is hydrogen or methyl.

2. The cosmetic for eyelashes according to claim 1, wherein the film-forming polymer (A) has a weight-average molecular weight of 5000 to 1000000.

3. The cosmetic for eyelashes according to claim 1 or 2, wherein the film-forming polymer (A) contains carboxyl groups which are partially or entirely neutralized.

4. The cosmetic for eyelashes according to claim 3, wherein the carboxyl groups are neutralized with ammonia and/or 2-amino-2-methyl-1-propanol as a neutralizing agent.

5. The cosmetic for eyelashes according to claim 3 or 4, wherein the degree of neutralization of the carboxyl groups contained in the film-forming polymer (A) is from 0.01 to 0.6.

6. The cosmetic for eyelashes according to any one of claims 1 to 5, wherein a homopolymer of the monomer (b) has a glass transition temperature (Tg₂) of 80°C or higher.

7. The cosmetic for eyelashes according to any one of claims 1 to 6, further comprising an oil component which is kept in a solid state at 25°C.

8. The cosmetic for eyelashes according to claim 7, wherein the oil component contains a wax having a rate of penetration of 8 or more.

9. The cosmetic for eyelashes according to any one of claims 1 to 8, further comprising a powder having an average particle size of from 1 to 20 µm.

10. The cosmetic for eyelashes according to claim 9, wherein the powder is an inorganic powder.

11. The cosmetic for eyelashes according to any one of claims 1 to 10, further comprising water and/or a lower alcohol having 1 to 4 carbon atoms to form an oil-in-water type emulsion composition.

12. The cosmetic for eyelashes according to any one of claims 1 to 11, further comprising a film-forming polymer emulsion.

13. The cosmetic for eyelashes according to claim 12, wherein a polymer contained in the polymer emulsion has a glass transition temperature (Tg₃) of from -25 to 15°C.

14. The cosmetic for eyelashes according to claim 12 or 13, wherein the polymer emulsion is synthesized by using sodium N-stearoyl-N-methyl taurine as an emulsifying agent.

## Patentansprüche

1. Kosmetikum für Augenwimpern, umfassend 1 bis 10 Gew.-%, bezogen auf das Gesamtgewicht des Kosmetikums, eines filmbildenden Polymers (A), umfassend 20 bis 90 Gew.-% eines Monomers (a), worin das Monomer (a) Konstitutionseinheiten sind, die von Methacrylsäure und/oder einem Salz davon stammen, und ein Monomer (b) mit einer Löslichkeit von 2 g oder weniger in 100 g Wasser, gemessen bei 20°C, und mit einer Glasübergangstemperatur (Tg₁) von 150 bis 230°C,
worin das Monomer (b) ausgewählt ist aus zumindest einem oder mehreren Monomeren, ausgewählt aus der Gruppe bestehend aus Methylmethacrylat, tert-Butylmethacrylat, tert-Butylacrylat, Isobornylacrylat, Isobornylmethacrylat, dargestellt durch die allgemeine Formel (1), Dicyclopentanylacrylat, Dicyclopentanylmethacrylat, dargestellt durch die allgemeine Formel (2), Styrol, tert-Butylacrylamid und tert-Butylmethacrylamid 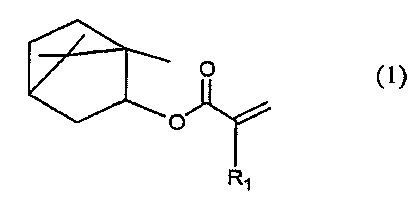 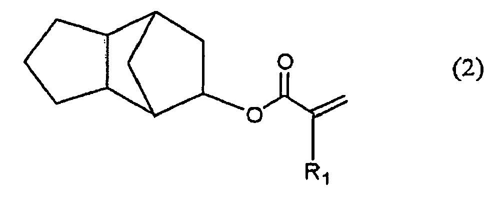 worin R₁ Wasserstoff oder Methyl ist.

2. Kosmetikum für Augenwimpern nach Anspruch 1, worin das filmbildende Polymer (A) ein Molekulargewicht im Gewichtsmittel von 5000 bis 1000000 hat.

3. Kosmetikum für Augenwimpern nach Anspruch 1 oder 2, worin das filmbildende Polymer (A) Carboxylgruppen enthält, die teilweise oder vollständig neutralisiert sind.

4. Kosmetikum für Augenwimpern nach Anspruch 3, worin die Carboxylgruppen mit Ammoniak und/oder 2-Amino-2-methyl-1-propanol als Neutralisierungsmittel neutralisiert sind.

5. Kosmetikum für Augenwimpern nach Anspruch 3 oder 4, worin der Neutralisationsgrad der Carboxylgruppen, die im filmbildenden Polymer (A) enthalten sind, von 0,01 bis 0,6 ist.

6. Kosmetikum für Augenwimpern nach einem der Ansprüche 1 bis 5, worin ein Homopolymer des Monomers (b) eine Glasübergangstemperatur (Tg₂) von 80°C oder mehr hat.

7. Kosmetikum für Augenwimpern nach einem der Ansprüche 1 bis 6, weiterhin umfassend eine Ölkomponente, die in einem festen Zustand bei 25°C gehalten ist.

8. Kosmetikum für Augenwimpern nach Anspruch 7, worin die Ölkomponente ein Wachs mit einer Eindringrate von 8 oder mehr enthält.

9. Kosmetikum für Augenwimpern nach einem der Ansprüche 1 bis 8, weiterhin umfassend ein Pulver mit einer durchschnittlichen Teilchengröße von 1 bis 20 µm.

10. Kosmetikum für Augenwimpern nach Anspruch 9, worin das Pulver ein anorganisches Pulver ist.

11. Kosmetikum für Augenwimpern nach einem der Ansprüche 1 bis 10, weiterhin umfassend Wasser und/oder einen niedrigen Alkohol mit 1 bis 4 Kohlenstoffatomen, zur Erzeugung einer Emulsionszusammensetzung vom Öl-in-Wasser-Typ.

12. Kosmetikum für Augenwimpern nach einem der Ansprüche 1 bis 11, weiterhin umfassend eine Filmbildungspolymeremulsion.

13. Kosmetikum für Augenwimpern nach Anspruch 12, worin ein Polymer, das in der Polymeremulsion enthalten ist, eine Glasübergangstemperatur (Tg₃) von -25 bis 15°C hat.

14. Kosmetikum für Augenwimpern nach Anspruch 12 oder 13, worin die Polymeremulsion durch Verwendung von Natrium-N-stearyl-N-methyltaurin als Emulgator synthetisiert ist.

## Revendications

1. Produit cosmétique pour les cils comprenant 1 à 10% en poids, sur la base du poids total du produit cosmétique, d'un polymère filmogène (A) contenant 20 à 90% en poids d'un monomère (a), dans lequel le monomère (a) est composé d'unités constitutionnelles dérivées de l'acide méthacrylique et/ou de l'un de ses sels, et un monomère (b) ayant une solubilité, mesurée à 20°C, de 2 g ou moins dans 100 g d'eau, et ayant une température de transition vitreuse (Tg₁) allant de 150 à 230°C,
dans lequel le monomère (b) est choisi parmi au moins un ou plusieurs monomères choisis dans le groupe constitué de méthacrylate de méthyle, méthacrylate de tert-butyle, acrylate de tert-butyle, acrylate d'isobornyle, méthacrylate d'isobornyle, représenté par la formule générale (1), acrylate de dicyclopentanyle, méthacrylate de dicyclopentanyle, représenté par la formule générale (2), styrène, acrylamide de tert-butyle, et méthacrylamide de tert-butyle 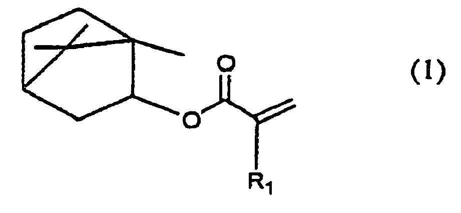 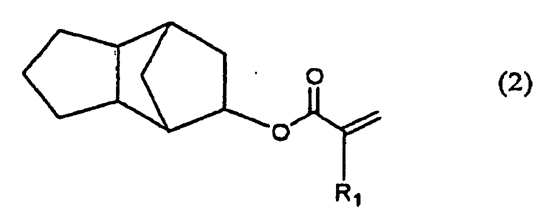 où R₁ est un atome d'hydrogène ou un groupe méthyle.

2. Produit cosmétique pour les cils selon la revendication 1, dans lequel le polymère filmogène (A) a une masse moléculaire moyenne en poids allant de 5000 à 1000000.

3. Produit cosmétique pour les cils selon la revendication 1 ou 2, dans lequel le polymère filmogène (A) contient des groupes carboxyliques qui sont partiellement ou totalement neutralisés.

4. Produit cosmétique pour les cils selon la revendication 3, dans lequel les groupes carboxyliques sont neutralisés par l'ammoniac et/ou le 2-amino-2-méthyl-1-propanol en tant qu'agent neutralisant.

5. Cosmétique pour les cils selon la revendication 3 ou 4, dans lequel le degré de neutralisation des groupes carboxyliques contenus dans le polymère filmogène (A) va de 0,01 à 0,6.

6. Cosmétique pour les cils selon l'une quelconque des revendications 1 à 5, dans lequel un homopolymère du monomère (b) présente une température de transition vitreuse (Tg₂) de 80°C ou plus.

7. Cosmétique pour les cils selon l'une quelconque des revendications 1 à 6, comprenant en outre un composant huileux qui est maintenu à l'état solide à 25°C.

8. Cosmétique pour les cils selon la revendication 7, dans lequel le composant huileux contient une cire présentant un taux de pénétration de 8 ou plus.

9. Cosmétique pour les cils selon l'une quelconque des revendications 1 à 8, comprenant en outre une poudre ayant une taille moyenne des particules allant de 1 à 20 µm.

10. Cosmétique pour les cils selon la revendication 9, dans lequel la poudre est une poudre minérale.

11. Cosmétique pour les cils selon l'une quelconque des revendications 1 à 10, comprenant en outre de l'eau et/ou un alcool inférieur ayant 1 à 4 atomes de carbone pour former une composition d'émulsion de type huile dans l'eau.

12. Cosmétique selon l'une quelconque des revendications 1 à 11, comprenant en outre une émulsion polymère filmogène.

13. Cosmétique pour les cils selon la revendication 12, dans lequel un polymère contenu dans l'émulsion polymère a une température de transition vitreuse (Tg₃) de -25 à 15°C.

14. Cosmétique pour les cils selon la revendication 12 ou 13, dans lequel l'émulsion polymère est synthétisée en utilisant la N-stéaroyl-N-méthyltaurine de sodium en tant qu'agent émulsifiant.
